# EUROPEAN PATENT APPLICATION

(11) **EP 2 402 322 A1**
(43) Date of publication of application: **04.01.2012**
(21) Application number: 10006843.6
(22) Date of filing: 02.07.2010
(51) Int. Cl.: C07D 217/20, A61K 31/472, A61P 25/00

(54) **1,2,3,4-tetrahydroisoquinoline derivative and its use as orexin receptor antagonist**

(71) Applicant: Actelion Pharmaceuticals Ltd., 4123 Allschwil (CH)
(72) Inventor: Boss, Christoph, 4123 Allschwil (CH); Brisbare-Roch, Catherine, 4123 Allschwil (CH); Jenck, François, 4123 Allschwil (CH); Steiner, Michel, 4123 Allschwil (CH)
(74) Representative: Velker, Jörg

(57) **Abstract**

The invention relates to the compound (R)-2-[(S)-1-(3,4-Dimethoxy-benzyl)-6,7-dimethoxy-3,4-dihydro-1*H*-isoquinolin-2-yl]-*N*-isopropyl-2-phenyl-acetamide; and to its use as active ingredient in the preparation of pharmaceutical compositions. The invention also concerns related aspects including processes for the preparation of the compound, pharmaceutical compositions containing the compound and methods of treatment comprising administration of said compound to a human being.

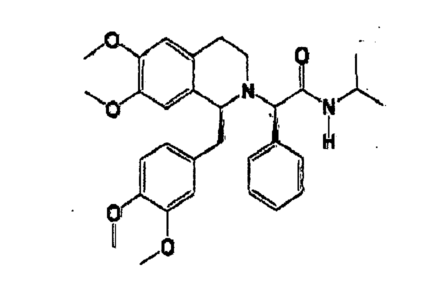

## Description

The present invention relates to the novel 1,2;3,4-tetrahydroisoquinoline derivative (R)-2-[(S)-1-(3,4-Dimethoxy-benzyl)6,7-dimethoxy-3,4-dihydro-1H-isoquinolin-2-yl]-N-isopropyl-2-phenyl-acetamide: 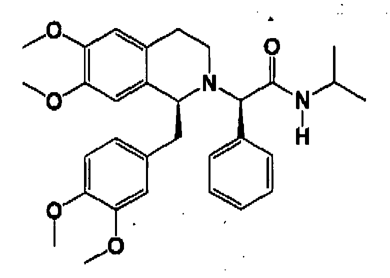 (hereinafter also referred to as COMPOUND) and its use as pharmaceutical. The invention also concerns related aspects including processes for the preparation of the compound, pharmaceutical compositions containing the compound, and its use as orexin receptor antagonist for diminishing disturbances resulting from stress exposure, or related to stress-associated psychiatric-disorders.

Orexins (orexin A or OX-A and orexin B or OX-B) are neuropeptides found in 1998 by two research groups; orexin A is a 33 amino acid peptide and orexin B is a 28 amino acid peptide (Sakurai T. et al., Cell, 92, (1998) 573-585). Orexins are produced in discrete neurons of the lateral hypothalamus and bind to the G-protein-coupled receptors (OX₁ and OX₂ receptors). The orexin-1 receptor (OX₁) is selective for OX-A, and the orexin-2 receptor (OX₂) is capable to bind OX-A as well as OX-B. OX-A may, therefore, be classified as a dual agonist of both orexin receptors. Orexins are found to regulate states of sleep and wakefulness opening potentially novel therapeutic approaches to narcolepsy as well as insomnia and other sleep disorders (Chemelli R.M. et al., Cell, 98, (1999) 437-451).

Orexin receptor antagonists (collectively referred to herein as "OXRA compounds") are a novel type of nervous system or psychotropic drugs. Their mode of action in animals and humans involves either blockade of both orexin-1 and orexin-2 receptor (dual antagonists), or individual and selective blockade of either the orexin-1 or the orexin-2 receptor (selective antagonists) in the brain.

The 1,2,3,4-tetrahydroisoquinoline derivative (2R)-2-{(1S)-6,7-dimethoxy-1-[2-(4-trifluoromethyl-phenyl)-ethyl]-3,4-dihydro-1H-isoquinolin-2-yl}-*N*-methyl-2-phenyl-acetamide (W0051118548), a dual orexin receptor antagonist, is currently in clinical development for primary insomnia. In the rat, the compound has been shown for example to cross the blood-brain barrier; to show a good systemic bioavailability; to decrease alertness, characterized by decreases in both active wake and locomotion; and to dose-dependently increase the time spent in both REM and NREM sleep (F. Jenck et al., Nature Medicine 13, (2007) 150-155). The compound has also been reported to be active in an animal model of conditioned fear: the rat fear potentiated startle paradigm (WO2009/0047723). However, when looking for an orexin receptor antagonist, which is suitable for diminishing disturbances resulting from stress exposure, or related to stress-associated psychiatric disorders, sleep-promoting effects such as decreased alertness and promotion of sleep, which are associated with dual orexin receptor blockade, may be undesired.

A particular 1,2,3,4-tetrahydroisoquinoline derivative is known from WO01/068609 and Chimia, 57, (2003), 270-275. The compound, 2-[1-(3,4-dimethoxy-banzyl)-6,7-dimethoxy-3,4-dihydro-1*H*-isoquinolin-2-yl]-*N*-butyl-2-phenyl-acetamide, which is disclosed in form of a mixture of diastereoisomeric racemates, is described to have an IC₅₀ on the orexin-1 receptor of 75 nM, and on the orexin-2 receptor of 1811 nM. When this compound, in form of its most active enantiomer: (R)-2-[(S)-1-(3,4-dimethoxy-benzyl)-6,7-dimethoxy-3,4-dihydro-1*H*-isoquinolin-2-yl]-*N*-butyl-2-phenyl-acetamide (hereinafter Reference Compound 1), which is a selective antagonist of the orexin-1, receptor, was tested for its potential to diminish disturbances resulting from stress exposure, or related to stress-associated psychiatric disorders; it showed at high dose no effect in the animal model of conditioned fear: the rat fear potentiated startle paradigm. In addition, Reference Compound 1 showed only moderate capacity to cross the blood-brain barrier.

It has now surprisingly been found that the novel 1,2,3,4-tetrahydroisoquinoline derivative: (R)-2-[(S)-1-(3,4-dimethoxy-benzyl)-6,7-dimethoxy-3,4-dihydro-1*H-*isoquinolin-2-yl]-*N-*isopropyl-2-phenyl-acetamide (COMPOUND), which is a selective antagonist of the orexin-1 receptor, is particularly suitable for diminishing disturbances resulting from stress exposure, or related to stress-associated psychiatric disorders. This compound crossed the blood-brain barrier and showed, surprisingly and contrary to Reference Compound 1, a significant effect in the animal model of conditioned fear: the rat fear potentiated startle paradigm.

In addition, the compound showed at relevant dose no effect in a model relevant to sleep induction. This compound may, therefore, in particular be useful as a medicament when sleep-promoting effects such as decreased alertness and promotion of sleep, which may be associated with drug treatment using a dual orexin receptor antagonist such as (2R)-2-{(1S)-6,7-dimethoxy-1-[2-(4-trifluoromethyl-phenyl)-ethyl]-3,4-dihydro-1H-isoquinolin-2-yl}-*N*-methyl-2-phenyl-acetamide, are not desired. This may for example be the case when the drug needs to be administered during daytime when sleep is not desired.
i) A first aspect of the invention relates to the novel 1,2,3,4-tetrahydroisoquinoline derivative (R)-2-[(S)-1-(3.4-Dimethoxy-benzyl)-6,7-dtmethoxy-3,4-dihydro-1*H*-isoquinolin-2-yl]-*N-*isopropyl-2-phenyl-acetamide (COMPOUND) 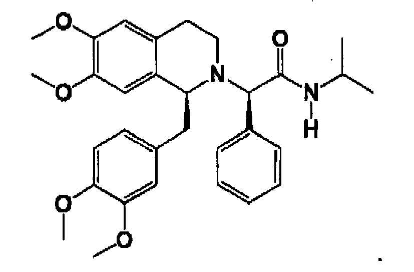
ii) According to a second embodiment of the invention, the COMPOUND according to embodiment i) may be used for the preparation of a medicament, and is suitable, for diminishing disturbances resulting from stress exposure (acute or chronic), or related to stress-associated psychiatric disorders. Such disturbances may be emotional, behavioural and/or physiological disturbances and may arise in combination or not with particular environmental conditions, or with a particular genetic predisposition, or a combination thereof.
iii) According to third embodiment, the COMPOUND is in particular suitable for the prevention or treatment of diseases or disorders mentioned herein, when sleep-promoting effects such as decreased alertness and promotion of sleep, (which effects may be considered to be related to treatment using, for example, a dual orexin receptor antagonist) are not desired.

Several lines of evidence demonstrate a role of the orexin system as modulator of the stress response. For instance, stress (i.e. psychological stress or physical stress) is associated with increased arousal and vigilance which in turn is controlled by orexins (Sutcliffe, JG, de Lecea, L; The hypocretins: setting the arousal threshold. Nat Rev Neurosci, 3(5) (2002) 339-349). Orexin neurons are likely to be involved in the coordinated regulation of behavioral and physiological responses in stressful environments (Kuru, M, Ueta, Y, Serino, R, Nakazato. M, Yamamoto, Y, Shibuya, I, Yamashita, H; Centrally administered orexin/hypocretin activates HPA axis in rats. Neuroreport, 11(9) (2000) 1977-1980). For instance, cardiovascular responses to conditioned fear and novelty exposure could be attenuated by a dual orexin receptor antagonist in rats (Furlong, TM, Vianna, DM, Liu, L, Carrive, P; Hypocretin/orexin contributes to the expression of some but not all forms of stress and arousal. Eur J Neurosci, 30(8) (2009) 1603-1614).

Stress response may lead to dramatic, usually time-limited physiological, psychological and behavioural changes that may affect appetite, metabolism and feeding behavior (Chrousos, GP, Gold, PW; The concepts of stress and stress system disorders. Overview of physical and behavioral homeostasis. JAMA, 267(9) (1992), 1244-1252). The acute stress response may include behavioural, autonomic and endocrinological changes, such as promoting heightened vigilance, decreased libido, increased heart rate and blood pressure, or a redirection of blood flow to fuel the muscles, heart and the brain (Majzoub, JA; Corticotropin-releasing hormone physiology European Journal of Endocrinology, 155 (suppl_1) (2006) 871-S76).

In addition to stress, the orexin system is also involved in appetitive/reward seeking behaviour (Berridge, CW, Espana, RA, Vittoz, NM; Hypocretin/orexin in arousal and stress. Brain Res, 1314 (2009), 91-102). In certain instances, a modulatory effect on stress may be complementary to an effect on appetitive/reward seeking behaviour as such. For instance, an OX₁ selective orexin receptor antagonist was able to prevent footshock stress induced reinstatement of cocaine seeking behaviour (Boutrel, B, Kenny, PJ, Specio, SE, Martin-Fardon, R, Markou, A, Koob, GF, de Lecea, L; Role for hypocretin in mediating stress-induced reinstatement of cocaine-seeking behavior. Proc Natl Acad Sci U S A, 102(52) (2005) 19168-19173). In addition, stress is also known to play an integral part in withdrawal which occurs during cessation of drug taking (Koob, GF, Zorrilla, EP; Neurobiological mechanisms of addiction: focus on corticotropin-releasing factor. Curr Opin Investig Drugs, 11(1) (2010) 63-71). Orexins have been found to increase food intake and appetite (Tsujino, N, Sakurai, J; Orexin/Hypocretin: a neuropeptide at the interface of sleep, energy homeostasis, and reward system. Pharmacol Rev, 61(2) (2009) 162-176). As an additional environmental factor, stress can contribute to binge eating behaviour, and lead to obesity (Adam, TC, Epel, ES; Stress, eating and the reward system. Physiol Behav, 91 (4) (2007) 449-458).

Further embodiments of the invention are presented hereafter:
iv) According to fourth embodiment, the COMPOUND according to any one of embodiments i) to iii) may be used for the preparation of a medicament, and is suitable, for diminishing disturbances resulting from stress exposure (acute or chronic).
v) In another embodiment, the COMPOUND according to any one of embodiments i) to iv) may be used for the preparation of a medicament, and is suitable, for diminishing addictions, anxiety disorders, affective disorders, eating disorders, or drinking disorders; resulting from stress exposure (acute or chronic), or related to stress-associated psychiatric disorders.
vi) In another embodiment, the COMPOUND according to any one of embodiments i) to iv) may be used for the preparation of a medicament, and is suitable, for diminishing stress-related syndromes.
vii) In another embodiment, the COMPOUND according to any one of embodiments i) to v) may be used to prepare a medicament, and is suitable, for diminishing addictions; resulting from stress exposure (acute or chronic). or related to stress-associated psychiatric disorders.

Especially, the COMPOUND is suitable to preventively or curatively treat patients who have been diagnosed as having an addiction, or as-being at risk of developing addictions; resulting from stress exposure (acute or chronic), or related to stress-associated psychiatric disorders; such as for example patients suffering from any form of depressive disorders, anxiety disorders, psychotic disorders, personality / dissociative disorders or eating disorders. In a sub-embodiment the COMPOUND is suitable to diminish addictions, notably to attenuate, decrease or prevent the occurrence of reinstatement of addiction, especially to drugs, resulting from stress exposure (acute or chronic), or under an environmental condition such as especially stress or fear.
viii) In another embodiment, COMPOUND according to embodiment i) may also be used for the preparation of a medicament, and is suitable, for diminishing addictions as such (notably for weakening the maintenance of addiction); wherein said addictions may be facilitated or precipitated by particular environmental conditions, or by a particular genetic predisposition, or by a combination thereof.
ix) In another embodiment, COMPOUND according to embodiment i) may also be used for the preparation of a medicament, and is suitable, for diminishing eating disorders as such, wherein said disorders may be facilitated or precipitated by particular environmental conditions, or by a particular genetic predisposition, or by a combination thereof.
x) In another embodiment, COMPOUND according to embodiment i) may also be used for the preparation of a medicament, and is suitable, for treating, or preventing anxiety disorders as such; wherein said disorders may be facilitated or precipitated by particular environmental conditions, or by a particular genetic predisposition, or by a combination thereof. In a sub-embodiment, COMPOUND according to embodiment i) may be used for the preparation of a medicament, and is suitable, for treating phobic anxieties (PHOBs) or post-traumatic stress disorders (PTSDs).

Any reference to COMPOUND according to embodiment i) is to be understood as referring also to the salts (and especially the pharmaceutically acceptable salts) of COMPOUND, as appropriate and expedient.

The term "pharmaceutically acceptable salts" refers to non-toxic, inorganic or organic acid and/or base addition salts. Reference can be made to "Salt selection for basic drugs", Int. J. Pharm. (1986), 33, 201-217.

The present invention also includes isotopically labelled. especially ²H (deuterium) labelled COMPOUND, which compounds are identical to COMPOUND except that one or more atoms have each been replaced by an atom having the same atomic number but an atomic mass different from the atomic mass usually found in nature. Isotopically labelled, especially ²H (deuterium) labelled COMPOUND and salts thereof are within the scope of the present invention. Substitution of hydrogen with the heavier isotope ²H (deuterium) may lead to greater metabolic stability, resulting e.g. in increased *in-vivo* half-life or reduced dosage requirements, or may lead to reduced inhibition of cytochrome P450 enzymes, resulting e.g. in an improved safety profile. In one embodiment of the invention, the COMPOUND is not isotopically labelled, or it is labelled only with one or more deuterium atoms. In a sub-embodiment, the COMPOUND is not isotopically labelled at all. Isotopically labelled COMPOUND may be prepared in analogy to the methods described hereinafter, but using the appropriate isotopic variation of suitable reagents or starting materials.

Where the plural form is used for compounds, salts, pharmaceutical compositions, diseases or the like, this is intended to mean also a single compound, salt. disease or the like.

The COMPOUND and pharmaceutically acceptable salts thereof can be used as medicaments, e.g. in the form of pharmaceutical compositions for enteral or parenteral administration.

A further aspect of the invention is a pharmaceutical composition containing COMPOUND, or a pharmaceutically acceptable salt thereof, and a pharmaceutically acceptable carrier material.

The production of the pharmaceutical compositions can be effected in a manner which will be familiar to any person skilled in the art (see for example Mark Gibson, Editor, Pharmaceutical Preformulation and Formulation. IHS Health Group, Englewood, CO, USA, 2001; Remington, The Science and Practice of Pharmacy, 20th Edition, Philadelphia College of Pharmacy and Science) by bringing the described COMPOUND or a pharmaceutically acceptable salt thereof, optionally in combination with other therapeutically valuable substances, into a galenical administration form together with suitable, non-toxic, inert, therapeutically compatible solid or liquid carrier materials and, if desired, usual pharmaceutical adjuvants.

The term "disturbances" as used in relation to the pharmacological use of COMPOUND as described herein may be defined as comprising all emotional, behavioural and/or physiological disturbances resulting from stress exposure (acute or chronic), or related to stress-associated psychiatric disorders. For instance, such disturbances comprise stress-related syndromes as such. In addition, the term "disturbances" comprises disturbances such as addictions, anxiety disorders, affective disorders, eating disorders, and drinking disorders. In a particular sub-embodiment, said disturbances may especially be defined as including eating disorders and, notably, addictions. In another particular sub-embodiment, said disturbances may especially be defined as including anxiety disorders.

The expression "diminishing" disturbances is to be understood as comprising any form of preventive or curative treatment (i.e. prevention or treatment) of such disturbances.

The expression "resulting from stress exposure (acute or chronic)" is relating to acute or chronic exposure to an "environmental condition" leading to stress / comprising a stress component. In particular, the expression means exposure to the "environmental conditions" fear or stress as such.

The expression "related to stress-associated psychiatric disorders" is relating to psychiatric disorders mediated by an "environmental condition" leading to stress / comprising a stress component. In particular the expression means psychiatric disorders mediated by the "environmental conditions" fear or stress as such. Examples of such "stress-associated psychiatric disorders" are for instance depressive disorders, anxiety disorders, psychotic disorders, personality / dissociative disorders or eating disorders.

The term "environmental condition" refers to environmental factors in general. Examples of such environmental factors may be of social origin or of physical origin and may themselves result from environmental factors. Examples of such environmental conditions may be of physical origin such as hunger, heat, cold, and the like; or, more importantly for human beings such conditions may for example relate to life changes such as bereavement, divorce, loss of work, or to chronic illness. In addition, environmental conditions also relate to stress (including stress caused by fear) or fear as such. Stress or fear in turn may be of social origin (e.g. social stress) or of physical origin (e.g. physical stress) and may in turn result from environmental factors. "Environmental conditions" which are such that they lead to stress/fear or that they comprise a stress/fear component, form a particular embodiment.

Stress-related syndromes comprise all types of stress-related syndromes and may be defined as comprising all syndromes or diseases caused by stress (acute or chronic), such as mood disorders and functional Illnesses; including bipolar disorder, impulse control disorder, chronic pain, dysthymia, migraine, borderline personality disorder, ADHD. major depressive disorder, burnout, chronic fatigue syndrome, fibromyalgia, gastrointestinal dysfunction such as irritable bowel syndrome, cardiovascular disease such as hypertension, immunsuppression, and infertitly. In addition, stress related syndromes may also be associated with addictions, anxiety disorders, affective disorders, eating disorders, or drinking disorders.

Eating disorders or pathologically modified food intake may be defined as comprising anorexias, bulimias, cachexia, binge eating disorder, metabolic dysfunction, dysregulated appetite control, or compulsive obesities. In a sub-embodiment, eating disorders may be defined as comprising anorexia nervosa, bulimia, cachexia, binge eating disorder, or compulsive obesities. In a particular sub-embodiment, eating disorders as defined before may result from / or may be facilitated or precipitated by environmental factors such as notably stress; or disturbed appetite (attraction or aversion for food); altered energy balance (intake vs. expenditure): disturbed perception of food quality (high fat or carbohydrates, high palatability); or disturbed food availability (unrestricted diet or deprivation):

Animal models that are clinically relevant models of binge eating in humans are described for example in "The biology of binge eating": W. Foulds Mathes, K. A. Brownley, X. Mo, C. M. Bulik; Appetite 52 (2009) 545-553.

Drinking disorders may be defined as comprising polydipsias in psychiatric disorders and all other types of excessive fluid intake. In a particular sub-embodiment, drinking disorders as defined before may result from / or may be facilitated or precipitated by environmental factors such as notably stress.

Anxiety disorders can be distinguished by the primary object or specificity of threat, ranging from rather diffuse as in generalized anxiety disorder, to circumscribed as encountered in phobic anxieties (PHOBs) or post-traumatic stress disorders (PTSDs). Anxiety disorders may, thus, be defined as comprising generalized anxiety disorders (GAD), obsessive compulsive disorders (OCDs), posttraumatic stress disorders (PTSDs), panic anxiety disorders (PADs) including panic attacks, all types of phobic anxiety (PHOBs), social phobia, avoidance, somatoform disorders including hypochondriasis, and separation anxiety. In a sub-embodiment, particular examples of circumscribed threat induced anxiety disorders are phobic anxieties (PHOBs) or post-traumatic stress disorders (PTSDs).

Affective disorders may be defined as comprising major depressive disorder and bipolar disorder.

Human addiction is a set of complex and interrelated forms of exaggerated emotional- and behavioural reactions to pleasant, attractive and, thus, rewarding stimuli (of natural or synthetic origin). For example, addiction to drugs is a chronically relapsing disorder' characterized by compulsive drug use and loss of control in limiting drug intake. The same holds true and applies for addiction to other rewarding stimuli of either natural or synthetic origin. Diminishing addictions may be defined as diminishing (i.e. to attenuate, to decrease or to prevent the occurrence of), either curatively or preventively, addiction to one or more rewarding stimuli in human beings. Such rewarding stimuli may be of either natural or synthetic origin. Examples of such rewarding stimuli are drugs {of either natural or synthetic origin; such as cocaine, amphetamines (notably metamphetamine), opiates [of natural or (semi-)synthetic origin such as morphine or heroin], cannabis, ethanol, mescaline, nicotine, and the like}, which drugs may be consumed alone or in combination; or other rewarding stimuli {of either natural origin (such as food, sweet, fat, or sex, and the like), or synthetic origin [such as gambling, or internet/IT (such as immoderate gaming (online gaming or video console playing)), or inappropriate Involvement in online social networking sites or blogging, and the like]}; wherein any group and any species of the above rewarding stimuli shall be understood as forming a particular sub-embodiment. Addiction comprises several stages including: (1) onset (preoccupation or anticipation); (2) binge or intoxication; (3) withdrawal or negative affect; (4) detoxification or abstinence; and (5) relapse or reinstatement; wherein any of the above stages shall be understood as forming a particular sub-embodiment; and wherein any of the above stages independently can be facilitated or precipitated by particular environmental conditions, or by a particular genetic predisposition, or by a combination thereof. In a particular sub-embodiment, one or several (notably one) of the stages of addiction as defined before result from / or are facilitated or precipitated by environmental conditions. Diminishing addiction notably refers to diminishing the onset of addictions, to weakening their maintenance, to facilitating withdrawal, to facilitating abstinence, or to attenuating, decreasing or preventing the occurrence of reinstatement of addiction (especially to diminishing the onset of addictions, to facilitating withdrawal, or to attenuating, decreasing or preventing the occurrence of reinstatement of addiction); under any environmental condition or genetic predisposition. Such help to diminish addiction may be applied on a short, middle and/or long term duration of treatment [i.e. as acute treatment, as sub-chronic treatment (such as treatment for one to up to four weeks), or as chronic treatment], wherein each of said duration terms of treatment forms a particular sub-embodiment. A particular sub-embodiment of diminishing addictions comprises attenuating, decreasing or preventing the occurrence of reinstatement of addiction, especially to drugs (such as especially amphetamines or notably cocaine), under an environmental condition such as especially stress or fear. Another particular sub-embodiment of diminishing addictions comprises diminishing the onset of addictions, notably to drugs such as especially morphine. Another particular sub-embodiment of diminishing addictions comprises facilitating abstinence, notably abstinence to drugs such as especially morphine.

The effect of a drug to diminish addiction may be modelled in normal or particularly sensitive mammals used as animal models [see for example Spealman et al, Pharmacol. Biochem. Behav. 64. 327-336 (1999); or T.S. Shippenberg, G.F. Koob, "Recent advances in animal models of drug addiction" in Neuropsychopharmacology: The fifth generation of progress; K.L.Davis, D. Charney, J.T.Doyle, C. Nemeroff (eds.) (2002); chapter 97, pages 1381-1397].

Another object of the present invention is a method of diminishing disturbances resulting from stress exposure (acute or chronic), or related to stress-associated psychiatric disorders in human beings; wherein such emotional, behavioural and/or physiological disturbances may arise in combination or not with particular environmental conditions, or a particular genetic predisposition, or a combination thereof; comprising the administration to a patient of a therapeutically effective amount of COMPOUND.

Another object of the present invention is the use of COMPOUND in combination with a sleep-inducing drug, such as for example a dual orexin receptor antagonist. Such combination may be useful in patients having, in addition to disorders mentioned herein, a disturbed sleep balance.

In a preferred embodiment of the invention, this amount of COMPOUND is comprised between about 1 mg and about 1000 mg per day, particularly between about 5 mg and about 500 mg per day, more particularly between about 50 mg and about 300 mg per day.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures. the term "about" placed before a temperature "Y" refers in the current application to an interval extending from the temperature Y minus 10°C to Y plus 10°C, and preferably to an interval extending from Y minus 5 °C to Y plus 5°C.

Whenever the word "between" is used to describe a numerical range, it is to be understood that the end points of the indicated range are explicitly included in the range. For example: if a temperature range is described to be between 40°C and 80°C, this means that the end points 40 °C and 80°C are included in the range or if a variable is defined as being an integer between 1 and 4, this means that the variable is the integer 1, 2, 3, or 4.

A further object of the invention is a process for the preparation of COMPOUND which is prepared according to the general sequence of reactions outlined in scheme 1 below. The COMPOUND obtained may also be converted into salts, especially pharmaceutically acceptable salts, thereof in a manner known *per se.*

As described in Scheme 1, the key-intermediates in the synthesis of COMPOUND is the 1-substituted 3,4-dihydroisoquinoline derivative (**4**).

The synthesis of the 1-substituted 3,4-dihydroisoquinoline starts by a condensation of homoveratrylamine (1, commercially available) with the appropriately substituted hydrocinnamic acid derivative (**2**) in the presence of a coupling reagent such as PyBOP and a base (e.g. DIPEA) in a solvent like DCM or DMF to give intermediate (**3**). Cyclization of the amide (**3**) to the 3,4-dihydroisoquinoline (**4**) is achieved by applying *Bischler-Napieralski* reaction-conditions (eg. POCl₃ in anhydrous MeCN at reflux for 2 h). Reduction of the 3,4-dihydroisoquinoline derivative (**4**) to the racemic 1,2,3,4-tetrahydroisoquinotine derivative (**5**) is achieved by switching the solvent from MeCN to MeOH, followed by the addition of NaBH₄. The final step in the synthesis of COMPOUND consists of an alkylation of the tetrahydroisoquinoline precursor (**5**) with the tosylate of mandelic acid-isopropyl amide (**7**) (prepared from mandelic acid (**6**) in analogy to the description given in WO2005/118548, p. 17) in a solvent such as 3-methyl-2-butanons in the presence of a base such as DIPEA at 80°C for 12 to 48h.

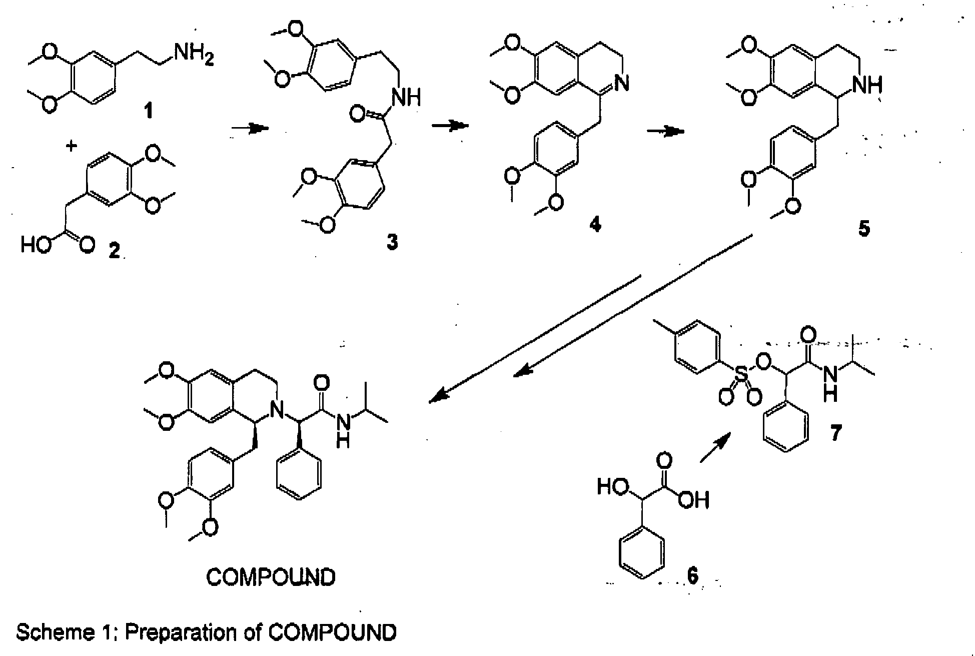

Whenever COMPOUND is obtained in the form of mixtures of stereoisomers, especially mixtures of epimers / enantiomers, the desired epimer / enantiomer can be isolated, using methods known to one skilled in the art: e.g. by formation and separation of diastereomeric salts or by HPLC over a chiral stationary phase such as a Regis Whelk-O1(R,R) (10 µm) column, a Daicel ChiralCel-OD-H (5-10 µm) column, or a Daicel ChiralPak lA (10 µm) or AD-H (5 µm) column. Typical conditions of chiral HPLC are an isocratic mixture of eluent A (EtOH, in presence or absence of an amine such as NEt₃, diethylamine) and eluent B (hexane), at a flow rate of 0.8 to 150 mL/min.

Alternatively, COMPOUND may be obtained in the desired enantiomerically enriched form by reduction of the 3,4-dihydroisoquinoline derivative (**4**) to the enantiomerically highly enriched 1,2,3,4-tetrahydroisoquinollne derivative (**5**) by hydrogenation using hydrogen gas or a hydrogen transfer compound (e.g. isopropanol, formic acid salts, or formic acid-NEt₃) in the presence of a chiral catalyst (chiral hydrogenation catalyst or transfer hydrogenation catalyst) and a solvent, and optionally in the presence of an additive.

Said catalysts are commercially available, prepared beforehand, or prepared in situ, from any commercially available Ru, Ir and Rh complex (also known as precursors), and a commercially available chiral ligand, chiral ligands, or a combination of different ligands, of which one has to be chiral. Suitable precursors are for example bis(2-methylallyl)(1.5cyclooetadione) Ruthenium, [RuCl₂(p-cymene)]₂, bis(1,5-cyclooctadiene) Iridium tetrafluoroborate, bis[chloro-1,5-cydooctadiefie-iridium], and bis(cyclooctadiene) Rhodium tetrafluoroborate. Preferred precursors are Ir-based precursors (Ru-based and Ir based precursors for the transferhydrogenation). Suitable chiral ligands are known by the person skilled in the art, and are for example described in the Handbook of Homogeneous Hydrogenation, J.G de Vries, C.J., Elsevier, Eds.; Wiley, 2007. chapter 23-35. Preferred chiral ligands are chiral bisphosphine ligands, and chiral monodentate phosphor containing ligands, amines, aminoalcohols or bisamines. Suitable chiral ligands are for example the bisphosphines, such as JosiPhos type ligands; MandyPhos; TaniaPhos type of ligands; BINAP, and its analogues, DUPhos; Chiraphos; and manodentate ligands such the MonoPhos type ligands, for example (3,5-dioxa4phosphacyclahepta[21-a;3,4aa']dinapthalen-4-yl) dimethylamine (MonoPhos).

Preferred hydrogenation procedures use the appropriate enantiomer of the commercially available TaniaPhos-ligand (1-dicyclohexylphosphino-2-[α-(dimethylamino)-2-(cyclohexyl-phosphino)bonzyl]-ferrocene) as chiral ligand. Alternatively, Noyori-type transfer hydrogenation, especially with a Ru-based chiral catalyst (R. Noyori et al; J. Am. Chem. Soc., 118, (1996) 4916-4917 and WO1997/020789) may be used with formic acid salts or the formic acid-NEt₃ complex as the hydrogen source.

The final step In the synthesis of COMPOUND consists of an alkylation of the enantiomerically highly enriched tetrahydroisoquinoline precursor (**5**) with the enantiomerically pure (S)-tosylate of mandelic acid-isopropyl amide (**7**) (prepared from enantiomerically pure(S)-mandelic acid (**8**) in analogy to the description given in WO2005/118548, p. 17) in a solvent such as 3-methyl-2-butanone in the presence of a base such as DIPEA at 80°C for 12 to 48h.

In case the racemic precursor (**5**) is alkylated with the enantiomerically pure (S)-tosylate of mandelic acid-isopropyl amide (**7**) the final product is obtained as mixture of diastereomers which can be separated by column chromatography to yield COMPOUND.

### Experimental section:

### Abbrevations (as used herein and in the description above):

- aq.: aqueous
- BSA: Bovine serum albumine
- CHO: Chinese hamster ovary
- DCM: dichloromethane
- DIPEA: n-ethyl-di-isopropylamine
- DMF: dimethylformamide
- DMSO: dimethylsulfoxide
- EDTA: ethylendiamine tetra acetic acid
- EtOAc: ethyl acetate
- EtOH: ethanol
- FC: flash column chromatography on silica gel
- FCS: Foatal calf serum
- FLIPR: Fluorescent imaging plate reader
- HBSS: Hank's balanced salt solution
- HEPES: 4-(2-hydroxyethyl)-piperazine-1-ethanesulfonic acid
- HPLC: high performance liquid chromatography
- LC: liquid chromatography
- M: molarity [mol L⁻¹]
- MeCN: acetonitrile
- MeOH: methanol
- MS: mass spectroscopy
- N: normality
- NEt₃: triethylamine
- org.: organic
- prep.: preparative
- PyBOP: Benzotriazole-1-yl-oxy-tris-pyrrolidine-phosphonium hexafluorophosphate
- rt: room temperature
- sat.: saturated
- TFA: trifluoroacetic acid
- THF: tetrahydrofuran
- Ts: tosyl, para-toluenesuffonyl
- TsO: tosylate, para-toluenesulfonate
- UV: ultraviolet

### I-Chemistry

All temperatures are stated in °C.

Compounds are characterized by:
LC-MS: Agilent 1100 series with DAD and MS detection (MS: Finnigan single quadrupole); columns (4.6x50 mm, 5 µm): Zorbax SB-AQ, Zorbax Extend C18; conditions: eluent A: MeCN, eluent B: TFA in water (0.4 mL/L). 5% to 95% MeCN, flow rate 4.5 mL/min; retention time (t_{R}) is given in min.

Compounds are purified by flash column chromatography on silica gel (FC) or by preparative HPLC using RP-C₁₈ based columns with MeCN/water gradients and ammonia additives.

### A. Preparation of precursors and intermediates:

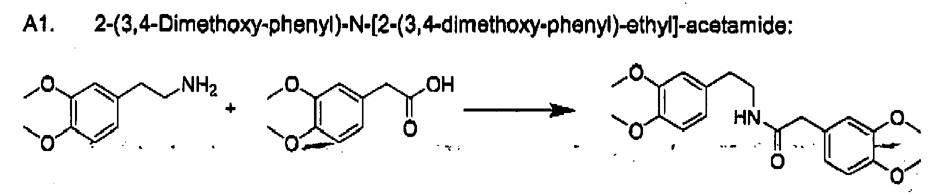

3,4-Dimethoxyphenylacetic acid (50 g; 250 mmol) were suspended in toluene (250 ml) followed by the addition of homoveratrylamine (46.7 g; 250 mmol). The suspension was heated to reflux with a *Dean-Stark* trap (while being heated up, the starting materials dissolved) and kept at reflux temperature for 12 h. The reaction mixture was then transferred into an Erlenmeyer flask containing toluene (300 ml) and cooled to rt while being stirred. The product precipitated and was filtered off and washed with toluene. The filter cake was dried at high vacuum to give 2-(3,4-dimethoxy-pheny)-N-[2-(3,4-dimethoxy-phenyl)-ethyt]-acetamide. LC-MS; t_{R} = 0.87 min; [M+H]⁺ = 360.26. 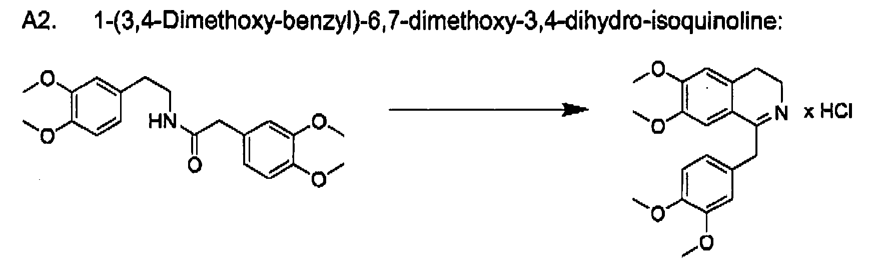 2-(3,4-Dimethoxy-phenyl)-N-[2-(3,4-dimothoxy-phenyl)-ethyl]-acetamide (30 g; 83.5 mmol) was dissolved in MeCN (400 ml) followed by the addition of POCl₃ (112 ml). The reaction mixture was put in a pre-heated oil bath (80°C) and stirred for 1 h. The reaction mixture was then slowly cooled to rt and stirring continued for 16 h. The solvents were evaporated under reduced pressure and MeOH was carefully added to the residue and evaporated again. This procedure was repeated three times before the crude product was dried at high vacuum to give 1-(3,4-dimethoxy-benzyl)-6,7-dimethoxy-3,4-dihydro-isoquinoline hydrochloride as a brown solid which was used in the next step without further purification. LC-MS: t_{R} = 0.75 min; [M+H]⁺ = 342.46.

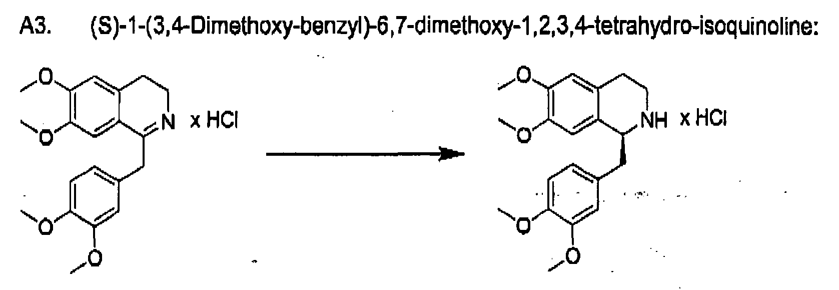

### Preparation of the hydrogenation catalyst:

N-(2-Amino-1,2-diphenyl-ethyl)2,4,6-trimethyl-benzenesulfonamide (468 mg; 1.186 mmol) was dissolved in MeCN (4 ml) followed by the addition of NEt₃ (0.668 ml; 4.789 mmol) and dichloro(p-cymene)ruthenium(II) (367 mg; 0.596 mmol). The reaction mixture was stirred at 80°C for 1 h and then cooled again to rt.

### Asymmetric reduction:

1-(3,4-Dimethoxy-benzyl)-6,7-dimethoxy-3,4-dihydro-isoquinoline hydrochloride (31 g: 83.5 mmol) was dissolved in dichloromethane (140 ml) followed by the addition of NEt₃ (35 ml) and the solution of the catalyst prepared as described above. To this mixture was added formic-acid-NEt₃ complex 5:2 (commercial available; 72 ml) and stirring under normal atmosphere was continued for 16 h followed by very careful addition of NaHCO₃ solution (250 ml) to the reaction mixture and extraction of the product with DCM (3x 250 ml). The combined org. layers were dried with MgSO₄, filtered and concentrated in vacuo. The crude product was dissolved again in dichloromethane (50 ml) followed by the addition of a solution of HCl in diethylether (1M; 75 ml). The solvents were evaporated and the residue was recrystallized from ethyl acetate to give (S)-1-(3,4-dimethoxy-benzyly)-6,7-dimethoxy-1,2,3,4-tetrahydro-isoquinoline as hydrochloride salt. LC-MS: t_{R} = 0.74 min; [M+H]^{*} = 344.12. 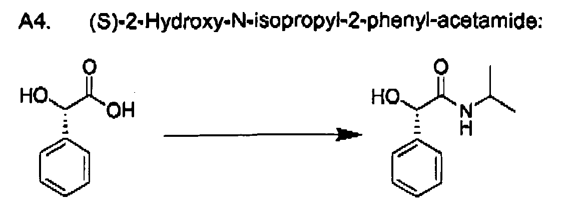 L-(+)-mandelic acid (20 g; 129 mmol) was dissolved in THF (400 ml) followed by the addition of isopropylamine (11.1 ml; 129 mmol) and N-hydroxysuccinimid (16.8 g;141.9 mmol). The reaction mixture was cooled to 0°C and N,N'-dicyclohexylcarbodiimide (29.6 g; 141.9 mmol) was added. The reaction mixture was slowly warmed to rt again and stirring continued for 48 h. The reaction mixture was filtered and the filtrate was concentrated under reduced pressure. The residue was dissolved in ethyl acetate and washed with NaHCO₃ solution (saturated), aqueous hydrochloric acid solution (1M) and brine. The org. layer was dried with MgSO₄, filtered and the solvent was evaporated under reduced pressure to give (S)-2-hydroxy-N-isopropyl-2-phenyl-acetamide. LC-MS: t_{R} = 0.71 min; [M+H]⁺ = 194.59. 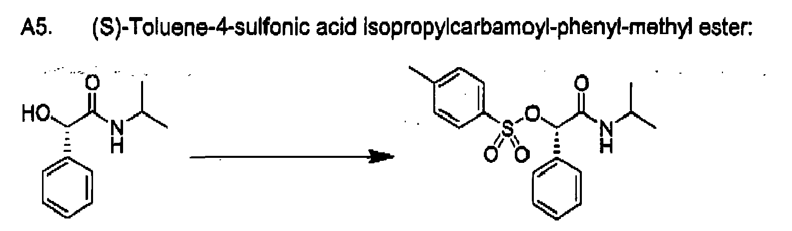 (S)-2-Hydroxy-N-isopropyl-2-phenyl-acetamide (24.9 g; 129 mmol) was dissolved in DCM (400 ml) and cooled to 0°C followed by the addition of NEt₃ (36 ml; 258 mmol), di-methylamino pyridine (1.84 g: 15.1 mmol) and toluene-4-sulfonylchloride (26 g; 136.6 mmol). Stirring was continued for 4 h while the reaction mixture was warmed to rt. followed by evaporation of the solvent under reduced pressure. The residue was dissolved in ethyl acetate and washed with CuSO₄ solution (saturated). The org layer was dried over MgSO₄, filtered and the solvent was evaporated under reduced pressure to give (S)-toluene-4-sulfonic acid isopropylcarbamoylphenyl-methyl ester. LC-MS: t_{R} = 1.02 min; [M+H]⁺ = 348.37.

### B. Examples:

**Example 1:** (R)-2-[(S)-1-(3,4-Dimethoxy-benzyl)-6,7-dimethoxy-3,4-dlhydro-1*H*-isoquinolin-2-yl]-*N*-isopropyl-2-phanyl-acetamide: 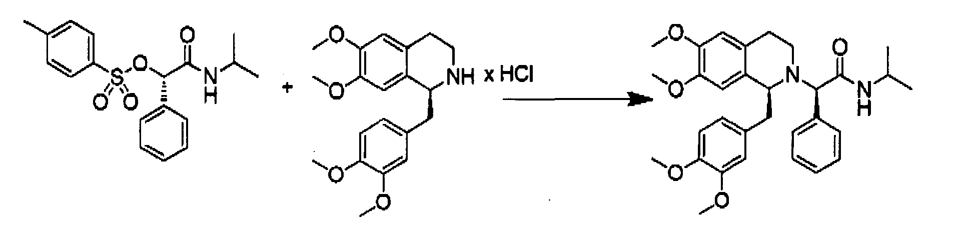 (S)-1-(3,4-Dimethoxy-benzyl)-6,7-dimethoxy-1,2,3,4-tetrahydro-isoquinoline hydrochloride (4.4 g; 11.6 mmol) was dissolved in methylethylketone (150 ml) and di-isopropylethylamine (4 ml; 23.2 mmol) followed by the addition of (S)-toluene-4-sulfonic acid isopropylcarbamoyl-phenylmethyl ester (8.06 g; 23.2 mmol). The reaction mixture was warmed to 70°C and stirring continued for 48 h. The mixture was cooled to rt and the solvent was evaporated under reduced pressure. The residue was dissolved in EtOAc and washed twice with brine. The org. layer was dried with MgSO₄, filtered and the solvent was evaporated under reduced pressure. The crude product was purified by FC (EtOAc / heptane = 2 / 1) to give (R)-2-[(S)-1-(3,4-dimethoxy-benzyl)-6,7-dimethoxy-3,4-dihydro-1*H*-isoquinolin-2-yl]-*N*-isopropyl-2-phenyl-acetamide as a foam. LC-MS: t_{R} = 0.88 min; [M+H]⁻ = 519.46.

**Reference Compound 1:** (R)-2-[(S)-1-(3,4-Dimethoxy-benzyl)-6,7-dimethoxy-3,4-dihydro-1*H-*isoquinolin-2-yl]-*N*-butyl-2-phenyl-acetamide:
The compound was prepared in analogy using (S)-toluene-4-sulfonic acid butylcarbamoylphenyl-methyl ester. LC-MS: t_{R} = 0.79 min; [M+H]⁺ = 533.46.

### II. Biological assays

### In vitro assay: intracellular calcium measurements:

Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % heat inactivated fetal calf serum (FCS). The cells are seeded at 20'000 cells / well into 384-well black clear bottom sterile plates (Greiner). The seeded plates are incubated overnight at 37°C in 5% CO₂.

Human orexin-A as an agonist is prepared as 1 mM stock solution in MeOH: water (1:1), diluted in HBSS containing 0.1 % bovine serum albumin (BSA), NaHCO₃: 0.375g/l and 20 mM HEPES for use in the assay at a final concentration of 3 nM.

Antagonists are prepared as 10 mM stock solution in DMSO, then diluted in 384-well plates using DMSO followed by a transfer of the dilutions into in HBSS containing 0.1 % bovine serum albumin (BSA), NaHCO₃: 0.375g/l and 20 mM HEPES. On the day of the assay, 50 µl of staining buffer (HBSS containing 1% FCS, 20 mM HEPES, NaHCO₃: 0.375g/l, 5 mM probenecid (Sigma) and 3 µM of the fluorescent calcium indicator fluo-4 AM (1 mM stock solution in DMSO, containing 10% pluronic) is added to each well. The 384-well cell-plates are incubated for 50 min at 37° C in 5% CO₂ followed by equilibration at RT for 30 - 120 min before measurement.

Within the Fluorescent Imaging Plate Reader (FLIPR Tetra, Molecular Devices), antagonists are added to the plate in a volume of 10 µl/well, incubated for 10 min and finally 10 µl/well of agonist is added. Fluorescence is measured for each well at 1 second intervals, and the height of each fluorescence peak is compared to the height of the fluorescence peak induced by 3 nM orexin-A with vehicle in place of antagonist. The IC₅₀ value (the concentration of compound needed to inhibit 50 % of the agonistic response) is determined and may be normalized using the obtained IC₅₀ value of a on-plate reference compound. Optimized conditions were achieved by adjustment of pipetting speed and cell splitting regime. The calculated IC₅₀ values may fluctuate depending on the daily cellular assay performance. Fluctuations of this kind are known to those skilled in the art.

COMPOUND has been measured on the orexin-1 receptor with an IC₅₀ value of 27 nM (geometric mean of n= 26 values).

COMPOUND has been measured on the orexin-2 receptor with an IC₅₀ value of > 500 nM (geometric mean of n= 26 values).

Reference Compound 1 has been measured on the orexin-1 receptor with an IC₅₀ value of 12 nM (geometric mean of n= 2 values).

Reference Compound 1 has been measured on the orexin-2 receptor with an IC₅₀ value of > 300 nM (geometric mean of n= 2 values).

### Measurement of brain and systemic concentration after oral administration:

In order to assess brain penetration, the concentration of the compound is measured in plasma ([P]), and brain .([B]), sampled 3 h (or at different time points) following oral administration (100 mg/kg) to male wistar rats. The compounds are formulated in 100% PEG 400. Samples are collected in the same animal at the same time point (+/- 5 min). Blood is sampled from the vena cava caudalis into containers with EDTA as anticoagulant and centrifuged to yield plasma. Brain is sampled after cardiac perfusion of 10 mL NaCl 0.9% and homogenized into one volume of cold phosphate buffer (pH 7.4). All samples are extracted with MeOH and analyzed by LC-MS/MS. Concentrations are determined with the help of calibration curves.

Results obtained for the compound of Example 1 (COMPOUND):
(3 h after oral administration (100 mg/kg), n = 3): [P] = 1460 ng / ml; [B] = 880 ng / g.

Results obtained for Reference Compound 1:
(3 h after oral administration (100 mg/kg), n = 3): [P] = 850 ng / ml; [B] = 530 ng / g.

### Absence of sleep-promoting effects:

### EEG, EMG and behavioural indices of alertness recorded by radiotelemetry in vivo in Wistar rats.

EEG and EMG signals were measured by telemetry using TL11 M2-F20-EET miniature radiotelemetric implants (Data Science Int.) with two pairs of differential leads.

Surgical implantation was performed under general anesthesia with Ketamin/Xylazln, for cranial placement of one differential pair of EEG electrodes and one pair of EMG leads inserted in either side of the muscles of the neck. After surgery, rats recovered in a thermoregulated chamber and received analgesic treatment with subcutaneous buprenorphine twice a day for 2 d. They were then housed individually and allowed to recover for a minimum of 2 weeks. Thereafter, rats—in their home cage—were placed in a ventilate sound-attenuating box, on a 12-h light / 12-h dark cycle, for acclimatization before continuous EEG / EMG recordings started. The telemetric technology that we used in this study allows accurate and stress-free acquisition of biosignals in rats placed In their familiar home cage environment, with no recording leads restricting their movements. Variables analyzed included four different stages of vigilance and sleep, spontaneous activity in the home cage and body temperature. Sleep and wake stages were evaluated using a rodent scoring software (Somnologica Science) directly processing electrical biosignals on 10 s contiguous epochs. The scoring is based on frequency estimation for EEG and amplitude discrimination for EMG and locomotor activity. Using these measurements, the software determines the probability that all components within each epoch best represent active waking (AW), quiet waking (QW), non-REM-sleep (NREM) or REM-sleep (REM). The percentage of total time spent in AW, QW. NREM- and REM-sleep was calculated per 12 h light or dark period. The latency to the onset of the first significant NREM- and REM-sleep episodes and the frequency and duration of those episodes were also calculated. AW, QW, NREM- and REM-sleep, home cage activity and body temperature were measured at baseline for at least one total circadian cycle (12 h-night, 12 h-day) before a test compound was administered. If baseline measurements indicated that animals were stable, test compound or vehicle was given in the evening by oral gavage at the end of the baseline 12- h day period, immediately before the nocturnal rise in orexin and activity in rats. All variables were subsequently recorded for 12 h following administration of the orexin receptor antagonist.

The compound of Example 1 has been tested in this assay (oral dosage: 300 mg/kg po): Results are: -4% on active wake, -3% on home cage activity, -2% on NREM sleep, +1% on REM sleep; when compared to vehicle controls.

### Generation of animal paradigms to test COMPOUND

There are a variety of tests in rodents that address fear and stress, as well as addictive syndromes that result from single or repeated exposure to synthetic or natural rewards, in combination or not with a particular environmental condition such as stress or fear in animals.

### Relapse to drug consumption

One significant barrier to successful treatment of drug addiction is that many detoxified drug abusers relapse into abuse during or after exiting treatment. It is likely that a significant percentage of relapses are precipitated by stress, exposure to environmental stimuli previously associated with drug, or by limited re-exposure to the abused drug itself (i.e., a "slip"). Currently, the most frequently used animal procedures modelling drug relapse are reinstatement procedures (e.g., see Epstein, DH, Preston, KL, Stewart, J, Shaham, Y; Toward a model of drug relapse: an assessment of the validity of the reinstatement procedure. Psychopharmacology (Berl), 189(1) (2006) 1-16). Rats will readily learn to press levers reinforced with intravenous drug (e.g. metamphetamine, nicotine, or especially cocaine) infusion. If, after having learned this response, lever pressing is no longer reinforced with drug infusion for several experimental sessions, responding will decrease to low rates (extinction). Reinstatement occurs when previously extinguished responding re-emerges as a result of an experimental manipulation.

One kind of experimental manipulation which can evoke reinstatement of previously-reinforced drug infusion is administering, independently of behavior, a period of mild intermittent footshock. In evaluating potential medications for treating drug relapse, a compound is administered preceding a test session in which responding is regularly reinstated with footshock. A result in which COMPOUND reduces response rates during such a test session, relative to when vehicle is administered, is consistent with the possibility that COMPOUND may have utility in preventing relapse to drug abuse, especially resulting from environmental conditions / stressors. An effect of COMPOUND observed in such a model may possibly be interpreted as a superimposed pharmacological effect of an anxiolytic / stress-reductive effect (as observed in the fear potentiated startle paradigm below) and an effect on addiction / drug craving as such (as observed in the behavioural (locomotor) sensitization models below).

Analogous protocols may be run with replacing footshock by non-contingent drug injection ("drug prime") or behavioural-contingent drug-associated cues ("chamber").

Methods: an infusion assembly system is connected to an infusion tether consisting of a 35 cm length of 0.40 mm i.d. polypropylene tubing encased within a 30 cm stainless steel spring to prevent damage. It is connected to a fluid swivel which is in turn attached via 0.40 polypropylene tubing to an infusion syringe. Indwelling venous catheters are implanted into the right external jugular vein under surgical anesthesia. Test chambers equipped with two retractable levers, a 5-w house light, and a tone generator are used. Infusion tubing is connected to an infusion pump that, when activated, delivered a 6-sec, 0.2 ml infusion. Recording of lever presses and activation of lights, shockers, pumps, and Sonalerts are accomplished by a microcomputer. Interface, and associated software.

### Conditioning to drug reward - Conditioned place preference

Conditioned place preference (CPP) is a widely used paradigm to study the rewarding effects of various drugs of abuse. Drug-induced CPP is based upon the principle that when a primary reinforcer (e.g., the drug) is paired with contextual stimuli, these contextual stimuli can acquire secondary reinforcing properties. These secondary reinforcing properties, which are presumably established due to a Pavlovian contingency, are thought to be capable of eliciting an operant place preference.

Method: In general, CPP is established by pairing a distinct set of contextual stimuli (visual, tactile, olfactory) with a drug. The drug is typically administered immediately prior to placing the animal into the context. In addition, animals are also given vehicle administrations paired with another set of contextual stimuli that are different from those paired with drug. Following conditioning (usually 2-4 exposures' to both drug and vehicle and their respective paired contexts), the animal, being in a drug-free state, is tested for place preference by being allowed free-choice access to the drug-paired and saline-paired contexts. CPP is then defined by a measure of preference (often time spent) for the drug paired context.

### Drug sensitization - Behavioural (locomotor) sensitization

Repeated administration of psychostimulants or opioids across various species including rodents causes the development of "reverse tolerance" known as sensitization. The term sensitization refers to an increase in a response (here locomotion) after the repeated occurrence of the stimulus (drug injection) that promoted the aforementioned response. The increased sensitivity to the locomotor stimulating effect of such drugs (behavioural sensitization) is believed to be relevant to the psychopathology, neurotoxicity and drug addiction and craving that develop in humans abusing psychostimulants.

Method: Although, in essence, locomotor sensitization is a non-associative learning process, the context plays a major role in the development and expression of locomotor sensitization. Thus, drug injections are usually administered intermittently in a context different from the home cage, such as an open field, where locomotor activity can be quantitatively measured. Drugs are regularly administered in high concentrations and via routes of administration with a fast onset of action (such as intraperitoneal or intravenous injections). In order to induce robust locomotor sensitization drugs are administered typically once a day over a period of several days. Once established in such a way, locomotor sensitization can be observed in response to a new drug challenge over a period of up to several months.

Effect_of COMPOUND on the acquisition of morphine induced locomotor sensitization

Method: Male Sprague Dawley rats are injected with morphine (10 mg/kg i.p.) and placed immediately afterwards in an open field (45 x 40 x 33 cm) for 45 min. Total distance moved (TDM) of the animal is recorded automatically via video-tracking. This procedure is repeated 4 times, every 2^{nd} day, so that each animal received a total number of 4 pairings of morphine injection within the context of the open field. Another group of rats is pre-treated with COMPOUND (300 mg/kg p.o.) 2 h before each morphine injection (pairing).

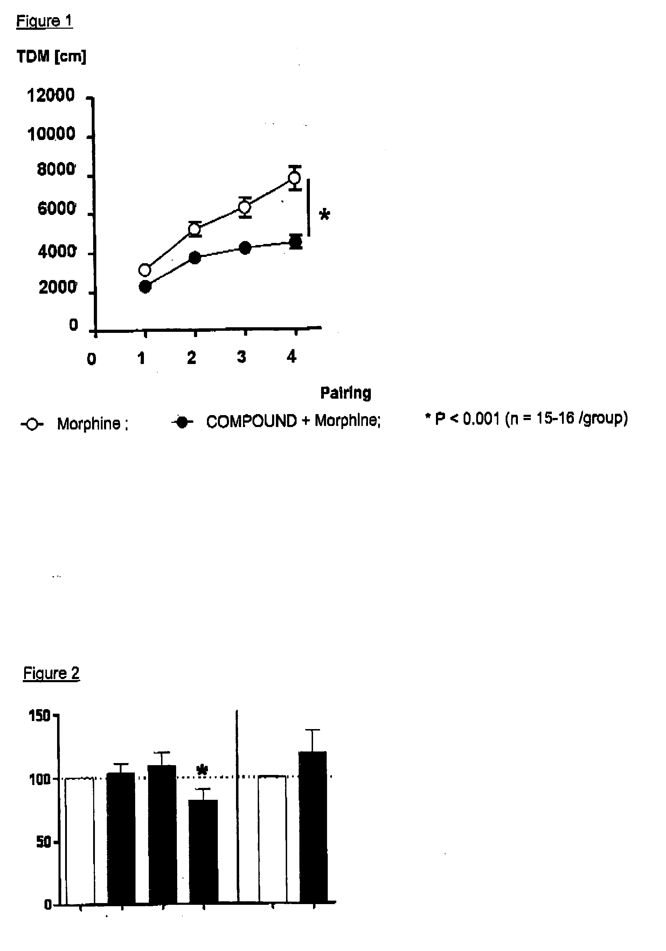

Results: Morphine injected rats developed,pronounced locomotor sensitization (i.e., their total distance moved after morphine injection at the fourth pairing was significantly greater than at the first pairing). Pre-treatment with COMPOUND significantly reduced this acquisition of locomotor sensitization to morphine (Figure 1).

### Figure 1. Effect of COMPOUND on the acquisition of morphine induced locomotor sensitization.

Figure 1 shows the total distance moved (TDM) after morphine injection at the first to fourth pairing.

Relevance of the finding: Sensitization to the locomotor effects of drugs of abuse is believed to reflect sensitization to the rewarding effects of these agents. For Instance, previous drug experience has been reported to increase the probability that animals will self-administer the drug later on. Thus, it is hypothesized that the extent to which sensitization to the rewarding effects of morphine develops in rats may reflect the extent of drug craving and, in consequence, reinstatement of compulsive drug-seeking behavior in humans. Accordingly, COMPOUND may have beneficial effects in treating opioid addicts early on in their addiction establishment by attenuating their drug craving (i.e., the "wanting" of the drug) and thereby reducing their likeliness towards later relapse of drug-seeking behavior. This effect may be complementary to the anxiolytic / stress-reducing effects observed in the fear potentiated startle paradigm below.

Effect of COMPOUND on the expression of morphine induced locomotor sensitization

Sensitization to the locomotor effects of drugs of abuse is believed to reflect sensitization to the rewarding effects of these agents. Thus, it is hypothesized that the extent to which sensitization to the rewarding effects of morphine persists in rats may reflect the extent of drug craving during abstinence.

Morphine injected rats develop a pronounced locomotor sensitization (i.e., their total distance moved after morphine injection at the fourth pairing was significantly greater than at the first pairing). A result in which pre-treatment with COMPOUND significantly reduces the expression of locomotor sensitization to morphine is consistent with the possibility that COMPOUND may have beneficial effects in treating drug (especially opioid) addicts during abstinence from the drug by attenuating their drug craving (i.e., the "wanting" of the drug) and thereby reducing their likeliness towards later relapse of drug-seeking behavior. This effect may be complementary to the anxiolytic / stress-reducing effects observed in the fear potentiated startle paradigm.

Method: Male Sprague Dawley rats are injected with morphine (10 mg/kg i.p.) and placed immediately afterwards in an open field (45 x 40 x 33 cm) for 45 min. Total distance moved (TDM) of the animal is recorded automatically via video-tracking. This procedure is repeated 4 times, every 2^{nd} day, so that each animal received a total number of 4 pairings of morphine injection within the context of the open field. 4 days after the last morphine injection rats are tested for the expression of locomotor sensitization. Half of the rats are pre-treated with vehicle the other half with COMPOUND (300 mg/kg p.o.) 2 h before they receive another morphine injection and are placed again in the open field.

### Fear-potentiated startle paradigm

The fear-potentiated startle (FPS) paradigm is a model of conditioned fear in rodents that relates to emotional states of fear and anxiety. Rats can be trained to associate an initially neutral, soon-to-be conditioned stimulus (CS: e.g. light) with an aversive, unconditioned stimulus (US; e.g. foot shook). When tested after training for their startle reflex response to brief (ms) noise bursts in the absence vs. the presence of the CS (e.g. light), rats generally show greater startle amplitudes during the presence of the CS which now serves as a predictor of shock. This increase in startle demonstrated during CS presentation vs. startle during absence of CS is referred to as FPS. This model translates to humans where a similar training procedure can be implemented and a FPS response can also be evoked in the presence of a shock predicting cue. Clinically efficacious anxiolytic drugs have been shown to reduce the startle reactivity under fearful conditions [cue (light) exposure] in both rodents and human (Davis, M, Falls, WA, Campeau, S, Kim, M; Fear-potentiated startle: a neural and pharmacological analysis. *Behav Brain Res,* 58(1-2) (1993), 175-198). The current paradigm represents a translational animal model that is predictive of anxiolytic activity of a drug.

Method: Sixteen adult male F344 rats were trained for two consecutive days (conditioning phase) to associate a light-stimulus with a foot shock (0.4 mA). On the following day they were tested for their response to startle eliciting noise stimuli which were delivered randomly under light (cue) or dark (no cue) conditions. One hour before the test, rats were treated (oral gavage) with vehicle (0.5% methylcellulose) or one dose of COMPOUND (30, 100, or 300 mg/kg). This procedure was repeated 4 times with a latin-square cross-over design allowing for a drug-washout phase of 1 week in between cross-over experiments. Average startle responses to noise stimuli of 90, 100 and 110dB intensity under light and dark conditions during the test day were measured and electrically transduced into a millivolt signal. This experiment was repeated under the same conditions (1h pretreatment, oral gavage, n=16 F344 rats, cross-over design) for vehicle (0.5% methylcellulose) and Reference Compound 1 (300 mg/kg). In order to compare both experiments, results were normalized to the respective vehicle group.

Results: The highest tested dose of COMPOUND (300 mg/kg) significantly reduced the fear-potentiated startle response under light (cue) conditions as compared to vehicle, whereas Reference Compound 1 at the same dose (300 mg/kg) had no effect (Figure 2).

Figure 2. Effects of COMPOUND and Reference Compound 1 on fear-potentiated startle responses under light (cus) conditions in F344 rats.

Figure 2 shows the startle amplitude (normalized to vehicle in %) for COMPOUND (bars on the left) and Reference Compound 1 (bars on the right). Startle amplitude is shown for vehicle p.o. (white bars); COMPOUND at the following dosages: 30mg/kg p.o. (light grey bar), 100 mg/kg p.o. (dark grey bar), and 300 mg/kg p.o. (black bar); and Reference Compound 1 at the dosage 300 mg/kg p.o. (black bar)

Relevance of the finding: Conditioned fear paradigms including fear-potentiated startle (FPS) mimic in particular the response to imminent specific threats and are, thus, suggested to model particularly the "fear component" of disturbances related to stress and/or anxiety disorders. Therefore, a potential anxiolytic effect of COMPOUND as revealed in the FPS paradigm may prove particularly beneficial for the treatment of disturbances resulting from stress exposure; such as for Instance relapse to drug abuse resulting from an acute environmental stress or fear factor. This effect may be complementary to any effect observed on addiction / drug craving in the behavioural (locomotor) sensitization models above.

## Claims

1. The compound (R)-2-[(S)-1-(3,4-Dimethoxy-benzy)-6,7-dimethoxy-3,4-dihydro-1*H-*isoquinolin-2-yl]-*N*-isopropyl-2-phonyl-acetamide 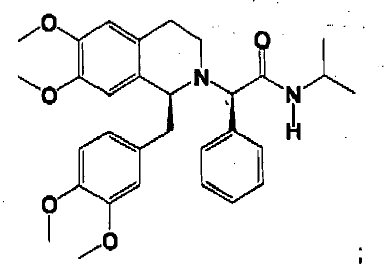 or a pharmaceutically acceptable salt thereof.

2. A pharmaceutical composition containing, as active principle, the compound according to claim 1, or a pharmaceutically acceptable salt thereof, and at least one therapeutically inert excipient.

3. The compound according to claim 1, or a pharmaceutically acceptable salt thereof, for use as a medicament.

4. The compound according to claim 1, or a pharmaceutically acceptable salt thereof, for diminishing disturbances resulting from stress exposure, or related to stress-associated psychiatric disorders.

5. Use of the compound according to claim 1, or of a pharmaceutically acceptable salt thereof, for the preparation of a medicament for diminishing disturbances resulting from stress exposure, or related to stress-associated psychiatric disorders.

6. The compound according to claim 4, or a pharmaceutically acceptable salt thereof, for diminishing addictions, anxiety disorders, affective disorders, eating disorders, or drinking disorders; resulting from stress exposure, or related to stress-associated psychiatric disorders.

7. The compound according to claim 4, or a pharmaceutically acceptable salt thereof, for diminishing stress-related syndromes.
